# EUROPÄISCHE PATENTSCHRIFT

(11) **EP 1 167 351 B1**
(45) Veröffentlichungstag und Bekanntmachung des Hinweises auf die Patenterteilung: **08.10.2003**
(21) Anmeldenummer: 01114524.0
(22) Anmeldetag: 16.06.2001
(51) Int. Cl.: C07D 207/26

(54) **Verfahren zur Abtrennung von Chlorwasserstoff aus einem N-Alkyl-2-pyrrolidon und Chlorwasserstoff enthaltenden Gemisch**
Process for the removal of hydrogen chloride from N-alkyl-2-pyrrolidone by azeotropic distillation
Procédé de séparation de chlorure d' hydrogène d'un mélange contenant du N-alkyl-2-pyrrolidone et du chlorure d'hydrogène

(30) Priorität: 27.06.2000 DE 10031288
(43) Veröffentlichungstag der Anmeldung: 02.01.2002
(73) Patentinhaber: Clariant GmbH, 65929 Frankfurt am Main (DE)
(72) Erfinder: Geissler, Holger, Dr., 55116 Mainz (DE)
(74) Vertreter: Mikulecky, Klaus, Dr.

(56) Entgegenhaltungen:
- EP-A- 0 949 256
- US-A- 3 399 119
- US-A- 3 651 166
- US-A- 3 658 659
- US-A- 4 276 126

## Beschreibung

Die vorliegende Erfindung betrifft ein Verfahren zur Abtrennung von Chlorwasserstoff aus einem ein N-Alkyl-2-pyrrolidon und Chlorwasserstoff enthaltenden Gemisch mittels Destillation.

Chlorwasserstoff enthaltendes N-Alkylpyrrolidon entsteht bei der Herstellung von Isochroman-3-onen nach dem in EP 0 949 256 beschriebenen Verfahren. Bei Verwendung von N-Methyl-2-pyrrolidon entsteht ein Chlorwasserstoff enthaltendes N-Methyl-2-pyrrolidon. Nach Abtrennung von Chlorwasserstoff kann das N-Methyl-2-pyrrolidon wieder in das Verfahren eingesetzt werden. Die Wiederverwendung des N-Methyl-2-pyrrolidons ist sowohl in ökonomischer Hinsicht als auch aus ökologischen Aspekten heraus von Vorteil. Durch den Wiedereinsatz wird die Entstehung unerwünschter Abfallprodukte, die entsorgt werden müssten, vermieden.

Die US 3,651,166 bezieht sich auf ein Verfahren zur Rückgewinnung von Halogenwasserstoff und organischen Basen, beispielsweise N-Methyl-2-pyrrolidon, durch Erhitzen ihrer Salze auf Dissoziationstemperatur. Beispiel II beschreibt die Rückgewinnung von wasserfreier HCI aus einer N-Methyl-2-pyrrolidon (NMP) Lösung, die Wasser und HCI enthält, durch Destillation. Hierbei wird zunächst bis zu einer Temperatur von ungefähr 120°C das Wasser abdestilliert. Dann wird ein Wasserrest und ein kleiner Teil des NMP bei 150°C abdestilliert. Die Abtrennung von wasserfreier HCI erfolgt anschließend bei 175 bis 180°C.

Auf diese Weise erhält man zwar wasserfreie HCI. Diese HCI ist jedoch in nicht unerheblichem Maße durch N-Methylpyrrolidon verunreinigt und lässt sich nicht unmittelbar weiterverarbeiten. Vielmehr muss diese verunreinigte HCI entweder in einem separaten, aufwendigen Prozess gereinigt werden oder gesondert entsorgt werden. Ferner können sich mitgeführte Feststoffe (Sublimate) im Kopf der Destillationskolonne und in den nachfolgenden Leitungen abscheiden und zu Verstopfungen und Blockaden führen. Siehe auch Vergleichsbeispiel 2A.

Es besteht daher ein Bedarf nach einem Verfahren, das die vorstehenden Nachteile vermeidet, sich auf einfache Art realisieren lässt, reinen Chlorwasserstoff liefert und eine ausreichende Abtrennung von Chlorwasserstoff von dem N-Alkyl-2-pyrrolidon ermöglicht.

Gelöst wird diese Aufgabe durch ein Verfahren zur Abtrennung von Chlorwasserstoff aus einem ein N-(C₁-C₁₈)-Alkyl-2-pyrrolidon und Chlorwasserstoff enthaltenden Gemisch, dadurch gekennzeichnet, dass man das ein N-(C₁-C₁₈)-Alkyl-2-pyrrolidon und Chlorwasserstoff enthaltende Gemisch bei 100 bis 220°C und 50 bis 850 hPa mittels einer Destillationskolonne in Gegenwart von Wasser destilliert, das Wasser als Wasser-Chlorwasserstoff-Azeotrop am Kopf unter Kühlen kondensiert , das WasserWasser-Chlorwasserstoff-Azeotrop in die Destillationskolonne zurückführt, über Kopf gasförmigen Chlorwasserstoff abtrennt und aus dem Sumpf das N-(C₁-C₁₈)-Alkyl-2-pyrrolidon entnimmt.

Das erfindungsgemäße Verfahren liefert überraschenderweise sowohl einen reinen Chlorwasserstoff, der beispielsweise in Wasser absorbiert und als wasserhaltiger Chlorwasserstoff weiterverwendet werden kann, als auch ein von Chlorwasserstoff weitestgehend befreites N-Alkyl-2-pyrrolidon, das ebenfalls ohne zusätzliche Aufarbeitung, beispielsweise in der Herstellung von Isochroman-3-onen, wieder eingesetzt werden kann.

Das erfindungsgemäße Verfahren besitzt zudem den Vorteil, dass es sich - ohne einen großen technischen Aufwand zu erfordern - auf einfache Weise realisieren lässt.

Unter N-Alkyl-2-pyrrolidonen werden Verbindungen der Formel 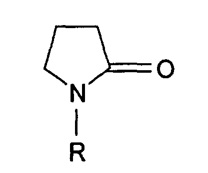 verstanden, worin R für einen geradkettigen oder verzweigten C₁-C₁₈-Alkylrest steht.

Die N-Alkyl-2-pyrrolidone bilden mit Chlorwasserstoff relativ stabile Addukte, die sich jedoch bei erhöhter Temperatur (Dissoziationstemperatur) in Chlorwasserstoff und das N-Alkyl-2-pyrrolidon spalten lassen. Die N-Alkyl-2-pyrrolidon-Chlorwasserstoff-Addukte neigen dazu, Sublimate zu bilden. Dies gilt insbesondere für das N-Methyl-2-pyrrolidon-HCI-Addukt, das vergleichsweise leicht sublimiert.

Ein weiterer Vorteil des erfindungsgemäßen Verfahrens besteht darin, dass die Ablagerungen von Feststoffen und die Abscheidung von Sublimaten, die Verstopfungen und Blockaden in den vom Kopf der Destillation weiterführenden Leitungen verursachen, überraschenderweise wirkungsvoll unterbunden werden.

Man kann das das N-Alkyl-2-pyrrolidon und HCI enthaltende Gemisch im Sumpf der Destillationskolonne vorlegen und erhitzen (diskontinuierliche Fahrweise). Man kann jedoch das das N-Alkyl-2-pyrrolidon und HCI enthaltende Gemisch auch dampfförmig oder flüssig der Destillationskolonne zu leiten und das erfindungsgemäße Verfahren kontinuierlich durchführen. Man setzt eine vorgegebene Menge Wasser zu, um zu gewährleisten, dass die Destillation in Gegenwart von Wasser abläuft und sich am Kopf der Destillationskolonne ein Wasser-Chlorwasserstoff-Azeotrop bilden kann. Arbeitet man kontinuierlich, so empfiehlt es sich das N-Alkyl-2-pyrrolidon und HCI enthaltende Gemisch der unteren Hälfte, insbesondere dem unteren Drittel der Destillationskolonne zuzuführen und aus dem Sumpf das von HCI weitestgehend befreite N-Alkyl-2-pyrrolidon in dem Maße, wie als Gemisch der Kolonne zugeleitet wird, abzuleiten.

Man stellt Druck und Temperatur so ein, dass das Chlorwasserstoff und das N-Alkyl-2-pyrrolidon enthaltende Gemisch siedet. Besonders günstig gestaltet sich das Verfahren, wenn man eine Temperatur wählt, die oberhalb der Sublimationstemperatur des N-Alkyl-2-pyrrolidon-HCI-Adduktes liegt. Die Sublimationstemperatur dieses N-Alkyl-2-pyrrolidon-HCI-Adduktes liegt oberhalb der Dissoziationstemperatur des Adduktes, also oberhalb der Temperatur, bei der das Addukt in HCI und das N-Alkylpyrrolidon zerfällt.

Am Kopf der Destillationskolonne fällt ein Dampfgemisch aus Wasser und Chlorwasserstoff an, das noch kleine Mengen von N-Alkyl-2-pyrrolidon enthalten kann. Das Wasser wird am Kopf der Kolonne kondensiert und fällt dabei als Wasser-Chlorwasserstoff-Azeotrop an, das noch untergeordnete Mengen N-Alkyl-2-pyrrolidon enthalten kann. Zugleich befindet sich am Kopf der Destillationskolonne überschüssiger gasförmiger Chlorwasserstoff. Dieser Chlorwasserstoff wird nicht zusammen mit dem Wasser als Chlorwasserstoff-Wasser-Azeotrop kondensiert, sondern entweicht gasförmig als reiner Chlorwasserstoff über Kopf. Eventuell noch mitgeführtes N-Alkyl-2-pyrrolidon bzw. organisches Produkt scheidet sich in flüssiger Form ab. Hierbei handelt es sich jedoch in der Regel um sehr geringe Mengen.

Das am Kopf unter Kühlung kondensierte Wasser-Chlorwasserstoff-Azeotrop wird in die Destillationskolonne zurückgeführt. Das Wasser verdampft erneut und scheidet sich wieder am Kopf der Destillationskolonne als Wasser-Chlorwasserstoff-Azeotrop ab und wird wieder in die Destillationskolonne zurückgeführt. Das Wasser wird auf diese Weise im Kreis geführt.

Da mit dem überschüssigen gasförmigen Chlorwasserstoff, der über Kopf abgeleitet wird, auch untergeordnete Mengen Wasser in Form von Wasserdampf entweichen, kann es notwendig werden, der Destillation bei längerem Betrieb Wasser in entsprechender Menge zuzusetzen, um die Bildung des Wasser-Chlorwasserstoff-Azeotrops und dessen Rückführung in die Destillation im gewünschten Umfang aufrechtzuerhalten.

Sollte das N-Alkyl-2-pyrrolidon und Chlorwasserstoff enthaltende Einsatzgemisch Wasser enthalten, so kann es bei längerem Betrieb der Destillation, insbesondere bei kontinuierlicher Destillation, erforderlich werden, Wasser in Form des Wasser-Chlorwasserstoff-Azeotrops am Kopf der Kolonne auszuschleusen, um die Wassermenge nicht unkontrolliert ansteigen zu lassen, sondern den Wasseranteil auf die für die Destillation erforderliche Menge zu begrenzen.

Man kann mit gutem Erfolg ein N-(C₁-C₁₂)-Alkyl-2-pyrrolidon und Chlorwasserstoff, insbesondere ein N-(C₁-C₈)-Alkyl-2-pyrrolidon und Chlorwasserstoff, bevorzugt ein N-(C₁-C₄)-Alkyl-2-pyrrolidon und Chlorwasserstoff, besonders bevorzugt ein N-Methyl-2-pyrrolidon und Chlorwasserstoff enthaltendes Gemisch einsetzen.

Wie eingangs bereits erwähnt, destilliert man bei einer Temperatur von 100 bis 220, insbesondere 120 bis 200, bevorzugt 130 bis 180°C.

Der Zusatz eines organischen Lösungsmittels ist nicht erforderlich, d.h. man führt die Destillation in Abwesenheit eines organischen Lösungsmittels durch.

Man führt, wie zuvor bereits genannt, die Destillation bei einem Druck 50 bis 850, insbesondere 100 bis 400, bevorzugt 150 bis 350 hPa (mbar) durch.

Das N-Alkyl-2-pyrrolidon und Chlorwasserstoff enthaltende Einsatzgemisch, enthält üblicherweise 0,1 bis 25, insbesondere 0,7 bis 15, bevorzugt 1,5 bis 10 Gew.-% HCI, bezogen auf Einsatzgemisch.

Man verwendet eine Destillationskolonne mit ausreichender Trennleistung. In vielen Fällen genügt es, eine Destillationskolonne mit 2 bis 40, insbesondere 4 bis 30, bevorzugt 5 bis 20 theoretischen Boden zu verwenden.

Man destilliert in Gegenwart von 0,05 bis 15, insbesondere 0,8 bis 10, bevorzugt 1 bis 8 Gew.-% Wasser, bezogen auf das im Sumpf und in der Destillationskolonne befindliche Gemisch. In vielen Fällen haben sich 2 bis 12, insbesondere 3 bis 10, bevorzugt 3,5 bis 8 Gew.-% Wasser, bezogen auf das im Sumpf und in der Destillationskolonne befindliche Gemisch, als ausreichend erwiesen.

Am Kopf der Destillationskolonne befindet sich ein Kühler, an dem sich das Wasser-Chlorwasserstoff-Azeotrop abscheidet. Man stellt am Kopf unter Kühlen üblicherweise eine Temperatur von -10 bis +60, insbesondere 0 bis 40, bevorzugt 5 bis 30°C ein. Bei diesen Temperaturen, die in gewissem Umfang auch vom eingestellten Druck abhängig sind und passend zu diesem Druck gewählt werden müssen, scheidet sich ein entsprechend diesen Destillationsbedingungen zusammengesetztes WasserWasser-Chlorwasserstoff-Azeotrop ab.

### Experimenteller Teil

### Beispiele:

### Beispiel 1

450 ml einer Mischung aus N-Methyl-2-pyrrolidon (NMP) und HCI (7,2 Gew.-% HCI) werden in einem beheizten Rührkolben, mit einer kleinen Kolonne, die mit Raschigringen gefüllt ist, vorgelegt. 10 ml Wasser werden vor dem Erhitzen zugegeben. Bei 200 mbar (hPa) stellt sich Rückfluss ein, die Kühlwassertemperatur des Kopfkühlers beträgt 10°C. Das kondensierte Wasser-Chlorwasserstoff-Azeotrop wird in die Destillationskolonne zurückgeführt. Die Sumpftemperatur wird dem Siedepunkt des NMP-HCI-Gemischs angepasst. Unter den Versuchsbedingungen sind das 150°C. Das entstehende HCI-Gas fällt in einer Reinheit von 99,3 % an und wird in einer separaten Waschkolonne in Wasser absorbiert. Es werden aus dem Sumpf regelmäßig Proben gezogen, die gegen NaOH 0,05 mol/l titriert werden, um den Fortschritt der HCI-Abtrennung zu verfolgen. Die ermittelten Werte sind der nachfolgenden Tabelle zu entnehmen.

| Zeit (min) | HCI Gew.-% |
|---|---|
| 0 | 7,2 |
| 15 | 6,76 |
| 30 | 5,81 |
| 45 | 4,7 |
| 60 | 4,04 |
| 75 | 3,45 |
| 90 | 2,9 |
| 120 | 2,29 |
| 135 | 1,94 |
| 180 | 1,56 |
| 230 | 1,37 |
| 310 | 1,3 |

### Vergleichsbeispiel 1

450 ml der in Beispiel 1 verwendeten Mischung aus N-Methylpyrrolidon (NMP) und HCI (7,2 Gew.-%) werden in der in Beispiel 1 benutzten Apparatur vorgelegt. 10 ml Wasser werden vor dem Erhitzen zugegeben. Bei 200 mbar (hPa) stellt sich Rückfluss ein, die Kühlwassertemperatur des Kopfkühlers wird jedoch auf 70°C eingestellt, um das Wasser abdestillieren zu können. Nach Abtrennen des Wassers, das als Wasser-Chlorwasserstoff-Azeotrop anfällt, beginnt sich ein NMP-HCI-Addukt im Kopfkühler abzuscheiden. Um ein Verstopfen des Kopfkühlers zu vermeiden, muss die Kühlwassertemperatur des Kopfkühlers auf 90°C erhöht werden. Die Sumpftemperatur wird dem Siedepunkt des NMP-HCI-Gemischs angepasst. Unter den Versuchsbedingungen sind das 150°C.
Das entstehende HCI-Gas fällt in einer Reinheit von lediglich 93,5 % an und wird in einer separaten Waschkolonne in Wasser absorbiert. Nach 330 Minuten wird die Destillation abgebrochen. Das verbleibende Reaktionsgemisch enthält 1,33 Gew.-% HCI.

### Beispiel 2

Die in diesem Versuch verwendete Laborglas-Kolonne, gefüllt mit Graphit Sulzerpackungen (BX Type, 1,5 m, Durchmesser 50 mm) besitzt einen 800ml Umlaufverdampfer und eine darin angeordnete Quarzheizkerze. Vor dem Start wird der Umlaufverdampfer mit der 800 ml einer Mischung aus NMP und 5 Gew.-% HCI und 50 ml Wasser gefüllt. Die Sumpftemperatur wird auf 160°C eingestellt. Ein vollständiger Rückfluss des Wasser-Chlorwasserstoff-Azeotrops stellt sich bei 200 mbar ein. Anschließend wird eine auf 150°C vorgewärmte Mischung aus NMP und 5 Gew.-% HCI kontinuierlich mit 200 g/h in das untere Drittel der Kolonne eingespeist. Nach 4 Stunden erhält man ein stabiles Temperaturprofil an der Kolonne und das kontinuierlich aus dem Sumpf ablaufende N-Methylpyrrolidon enthält lediglich 0,4 - 0,7 Gew.-% HCI. Die mittlere Verweilzeit für das N-Methylpyrrolidon beträgt 4 Stunden. Während des Versuches beträgt die Temperatur im Sumpf 160°C; die Kühlertemperatur 30°C und die Kopftemperatur 68°C und der Druck 200 mbar. Das entstehende HCI-Gas fällt in einer Reinheit von 97,8 % an und wird in einer nachgeschalteten Waschkolonne als wässrige 30 %ige HCI absorbiert.

### Beispiel 3

Der Versuch wird analog Beispiel 2 mit der Ausnahme, dass die Kühlertemperatur 10°C beträgt, durchgeführt. Das entstehende HCI-Gas fällt in einer Reinheit von 99,4 % an und wird in einer nachgeschalteten Waschkolonne als wässrige HCI absorbiert.

### Beispiel 4

Der Versuch wird analog Beispiel 2 mit der Ausnahme, dass die Kühlertemperatur -10°C beträgt, durchgeführt. Das entstehende HCI-Gas fällt in einer Reinheit von 99,8 % an und wird in einer nachgeschalteten Waschkolonne als wässrige HCI absorbiert.

### Vergleichsbeispiel 2A

Der Versuch wird analog Beispiel 2 mit der Ausnahme, dass vor dem Start der Umlaufverdampfer nicht mit 800 ml einer Mischung aus NMP und 5 Gew.-% HCI und 50 ml Wasser, sondern mit 850 ml einer Mischung aus NMP und 5 Gew.-% HCI jedoch ohne Wasser gefüllt wird, durchgeführt. Während des Versuches beträgt die Temperatur im Sumpf 160°C, die Kühlertemperatur 30°C und die Kopftemperatur 137°C und der Druck 200 mbar. Der Versuch muss nach ca. einer Stunde abgebrochen werden, da das sich fest absetzende Addukt aus N-Methylpyrrolidon und Chlorwasserstoff den Kühler verstopft.

### Vergleichsbeispiel 2B

Der Versuch wird analog Beispiel 2 mit der Ausnahme, dass vor dem Start der Umlaufverdampfer nicht mit 800 ml einer Mischung aus NMP und 5 Gew.-% HCI und 50 ml Wasser, sondern mit 850 ml einer Mischung aus NMP und 5 Gew.-% HCI gefüllt wird, durchgeführt. Während des Versuches beträgt die Temperatur im Sumpf 160°C, die Kühlertemperatur 65°C und die Kopftemperatur 137°C und der Druck 200 mbar. Die Kühlertemperatur wurde so gewählt, dass sich kein Feststoff absetzt. Das entstehende HCI-Gas fällt in einer Reinheit von 95,5 % an.

### Beispiel 5

200 g/h einer auf 150°C vorgewärmten Mischung aus NMP und 7 Gew.-% HCI, werden kontinuierlich in das untere Drittel der nachstehend genannten Kolonne eingespeist. Die in diesem Versuch verwendete Laborglas-Kolonne, gefüllt mit Graphit Sulzerpackungen (BX Type; 1,5 m, Durchmesser 50 mm) besitzt einen 800 ml Umlaufverdampfer und eine darin angeordnete Quarzheizkerze. Vor dem Start wird der Umlaufverdampfer mit der Mischung aus NMP und 7 Gew.-% HCI und 50 ml Wasser gefüllt. Ein vollständiger Rückfluss des Wasser-HCI-Azeotrops stellt sich bei 200 mbar ein. Die Kühlertemperatur beträgt 10°C. Über Kopf wird entstehendes HCI-Gas kontinuierlich entfernt und in einer nachgeschalteten Waschkolonne in Wasser absorbiert. Die Sumpftemperatur wird auf 160°C eingestellt. Das aus dem Sumpf ablaufende N-Methylpyrrolidon enthält 0,4 bis 0,7 Gew.-% HCI. Die mittlere Verweilzeit für das N-Methylpyrrolidon beträgt 4 Stunden.

## Patentansprüche

1. Verfahren zur Abtrennung von Chlorwasserstoff aus einem ein N-(C₁-C₁₈)-Alkyl-2-pyrrolidon und Chlorwasserstoff enthaltenden Gemisch, **dadurch gekennzeichnet, dass** man das ein N-(C₁-C₁₈)-Alkyl-2-pyrrolidon und Chlorwasserstoff enthaltende Gemisch bei 100 bis 220°C und 50 bis 850 hPa mittels einer Destillationskolonne in Gegenwart von Wasser destilliert, das Wasser als Wasser-Chlorwasserstoff-Azeotrop am Kopf unter Kühlen kondensiert , das Wasser-Chlorwasserstoff-Azeotrop in die Destillationskolonne zurückführt, über Kopf gasförmigen Chlorwasserstoff abtrennt und aus dem Sumpf das N-(C₁-C₁₈)-Alkyl-2-pyrrolidon entnimmt.

2. Verfahren nach Anspruch 1, **dadurch gekennzeichnet, dass** man ein ein N-(C₁-C₁₂)-Alkyl-2-pyrrolidon und Chlorwasserstoff enthaltendes Gemisch einsetzt.

3. Verfahren nach Anspruch 1 oder 2, **dadurch gekennzeichnet, dass** man ein Gemisch, das 0,1 bis 25 Gew.-% Chlorwasserstoff enthält, einsetzt.

4. Verfahren nach einem oder mehreren der Ansprüche 1 bis 3, **dadurch gekennzeichnet, dass** man bei 100 bis 400 hPa destilliert.

5. Verfahren nach einem oder mehreren der Ansprüche 1 bis 4, **dadurch gekennzeichnet, dass** man mittels einer Destillationskolonne mit 2 bis 40 theoretischen Böden destilliert.

6. Verfahren nach einem oder mehreren der Ansprüche 1 bis 5, **dadurch gekennzeichnet, dass** man in Gegenwart von 0,05 bis 15 Gew.-% Wasser, bezogen auf das im Sumpf und in der Destillationskolonne befindliche Gemisch, destilliert.

7. Verfahren nach einem oder mehreren der Ansprüche 1 bis 6, **dadurch gekennzeichnet, dass** man am Kopf unter Kühlen eine Temperatur von -10 bis +60°C einstellt.

## Claims

1. A process for separating hydrogen chloride from a mixture comprising an N-(C₁-C₁₈)-alkyl-2-pyrrolidone and hydrogen chloride, which comprises distilling the mixture comprising an N-(C₁-C₁₈)-alkyl-2-pyrrolidone and hydrogen chloride at from 100 to 220°C and from 50 to 850 hPa in the presence of water by means of a distillation column, condensing the water as water-hydrogen chloride azeotrope at the top by cooling, returning the water-hydrogen chloride azeotrope to the distillation column, separating off gaseous hydrogen chloride at the top and taking off the N-(C₁-C₁₈)-alkyl-2-pyrrolidone from the bottom.

2. The process as claimed in claim 1, wherein a mixture comprising an
N-(C₁-C₁₂)-alkyl-2-pyrrolidone and hydrogen chloride is used.

3. The process as claimed in claim 1 or 2, wherein a mixture containing from 0.1 to 25% by weight of hydrogen chloride is used.

4. The process as claimed in one or more of claims 1 to 3, wherein the distillation is carried out at from 100 to 400 hPa.

5. The process as claimed in one or more of claims 1 to 4, wherein the distillation is carried out by means of a distillation column having from 2 to 40 theoretical plates.

6. The process as claimed in one or more of claims 1 to 5, wherein the distillation is carried out in the presence of from 0.05 to 15% by weight of water, based on the mixture present in the bottom and in the distillation column.

7. The process as claimed in one or more of claims 1 to 6, wherein a temperature of from -10 to +60°C is set at the top by means of cooling.

## Revendications

1. Procédé pour la séparation de chlorure d'hydrogène à partir d'un mélange renfermant une N-(alkyl en C₁-C₁₈)-2-pyrrolidone et du chlorure d'hydrogène, **caractérisé en ce qu'**on distille un mélange renfermant une N-(alkyl en C₁-C₁₈)-2-pyrrolidone et du chlorure d'hydrogène, à une température de 100 à 220°C et une pression de 50 à 850 hPa, dans une colonne de distillation en présence d'eau, on condense l'eau comme azéotrope eau-chlorure d'hydrogène à la tête en refroidissant, on recycle l'azéotrope eau-chlorure d'hydrogène à la colonne de distillation, on sépare le chlorure d'hydrogène gazeux par la tête et on reprend la N-(alkyl en C₁-C₁₈)-2-pyrrolidone de la coupe de queue.

2. Procédé selon la revendication 1, **caractérisé en ce qu'**on utilise un mélange renfermant une N-(alkyl en C₁-C₁₂)-2-pyrrolidone et du chlorure d'hydrogène.

3. Procédé selon la revendication 1 ou 2, **caractérisé en ce qu'**on utilise un mélange qui renferme de 0,1 à 25 % en masse de chlorure d'hydrogène.

4. Procédé selon l'une des revendications 1 à 3, **caractérisé en ce qu'**on réalise la distillation à des pressions de 100 à 400 hPa.

5. Procédé selon l'une des revendications 1 à 4, **caractérisé en ce qu'**on distille en utilisant une colonne de distillation comprenant 2 à 40 plateaux théoriques.

6. Procédé selon l'une ou plusieurs des revendications 1 à 5, **caractérisé en ce qu'**on réalise la distillation en présence de 0,05 à 15 % en masse d'eau, par rapport au mélange se trouvant dans la coupe de queue et dans la colonne de distillation.

7. Procédé selon une ou plusieurs des revendications 1 à 6, **caractérisé en ce qu'**on établit à la tête en refroidissant une température de -10 à +60°C.
